Europäisches Patentamt

European Patent Office

Office européen des brevets

(19)

(11) Publication number: **0 412 762 A2**

# EUROPEAN PATENT APPLICATION

(12)

(21) Application number: 90308652.8

(22) Date of filing: 06.08.90

(51) Int. Cl.⁵: **C07K 7/06**, C07K 7/08, A61K 37/02

(30) Priority: 07.08.89 US 390569
09.04.90 US 506981

(43) Date of publication of application:
13.02.91 Bulletin 91/07

(84) Designated Contracting States:
AT BE CH DE DK ES FR GB IT LI LU NL SE

(71) Applicant: MERCK & CO. INC.
126, East Lincoln Avenue P.O. Box 2000
Rahway New Jersey 07065-0900(US)

(72) Inventor: Oliff, Allen I.
1412 Florence Drive
Gwynedd Valley, PA 19437(US)
Inventor: Riemen, Mark W.
5122 Briar Stone Trace
Carmel, Indiana 46032(US)

(74) Representative: Hesketh, Alan, Dr. et al
European Patent Department Merck & Co.,
Inc. Terlings Park Eastwick Road
Harlow Essex, CM20 2QR(GB)

(54) Peptide inhibitors of human papilloma virus protein binding to retinoblastoma gene proteins.

(57) The present invention comprises biochemically pure polypeptides, identified from the Human Papilloma Virus (HPV) E7 protein, which efficiently block the binding of the HPV E7 protein to the protein encoded by the retinoblastoma gene (RBG). The polypeptides are antagonists of the biochemical interaction of the HPV E7 protein and the RBG protein. The invention is useful in the treatment of genital warts and cervical cancer. One polypeptide of the present invention (Peptide A) has an amino acid sequence of:
Thr-Asp-Leu-Tyr-Cys-Tyr-Glu-Gln-Leu-Asn-Asp-Ser-Ser.

EP 0 412 762 A2

# PEPTIDE INHIBITORS OF HUMAN PAPILLOMA VIRUS PROTEIN TO RETINOBLASTOMA GENE PROTEIN

## BACKGROUND OF THE INVENTION

Human Papilloma Viruses (HPVs) are small DNA containing viruses. HPVs are classified as members of the papovavirus family along with similar small DNA containing viruses like Simian Virus 40 (SV40) and Polyoma Virus. Over two dozen genetically distinct strains of HPV have been isolated and are classified by DNA sequence homology using hybridization techniques. These are referred to as HPV-1, HPV-2, etc. The various strains appear to be responsible for different disease states. HPVs are of medical interest because they are responsible for or contribute to the developement of a variety of epithelial cell proliferative diseases in man. For example, infection with HPV strains 16 and 18 is associated with the development of cancer of the cervix. It has been postulated that HPV acts as an initiator in cervical carcinogenesis and that malignant transformation depends on interaction with other factors. Infections with BPV strains 6 and 11 is associated with the development of genital warts. The incidence of HPV infection appears to be increasing as shown by a large increase recently in patient visits related to genital HPV infections in both males and females and the presence of HPV in pap smears of some women under 30 years of age.

The nature of HPV-16 in particular and papilloma viruses in general has been well studied recently. HPV-16 contains a 7904 bp double-stranded DNA genome (Siedorf, K., et al, Virology (1985) 145: 181-185). The capsid is 50 nm and contains 72 capsomers (Klug, A., J Mol Biol (1965) 11: 403-423). Additionally, U.S. Patent 4,777,239 discloses a series of seventeen synthetic peptides which are said to be capable of raising antibodies to HPV-16 and thus may be useful for diagnostic purposes.

The DNAs of several papilloma viruses have been sequenced, including several HPV types, bovine papilloma virus (BPV) and cottontail rabbit papilloma virus (CRPV). All of these display similar patterns of nucleotide sequence with respect to open reading frames. The open reading frames can be functionally divided into early region (E) and late regions (L); the E regions are postulated to encode proteins needed for replication and transformation; and the L regions to encode the viral capsid proteins (Danos, O., et al. J. Invest Derm (1984) 83: 7s-11s).

Two HPV encoded proteins, E6 and E7, are thought to be involved in the pathogenesis of HPV induced abnormal cell proliferation. The amino acid sequence of the HPV-16 E7 protein as deduced from the nucleic acid sequence is shown in N. Salzman and P. Howley, "The Papovaviridae", Vol. 2, p. 379, Plenum Press, N.Y. (1987).

The HPV genes encoding the E6 and E7 proteins are invariably expressed in tissue or tumor cells obtained from cervical cancers associated with HPV infection. In addition, the HPV E6 and E7 genes derived from the HPV-16 strain are capable of inducing epithelial cell transformation in cell culture without the presence of other HPV genes. These observations indicate that at least part of the stimulation of cell proliferation caused by HPV infection is due to the E6 and E7 viral proteins.

The HPV E7 protein has been shown to bind to the retinoblastoma gene (RBG) encoded protein. The RBG has been shown to participate in growth control of some types of human cancers. Specifically, mutations which inactivate the RBG are associated with abnormal or increased cell proliferation. Morover, introduction of the normal RBG into tumor cells that are missing the full length RBG results in decreased cell proliferation and reduced capacity to form tumors in animals. Thus, binding of the HPV E7 protein to the RBG protein, which itself is a known regulator of cell growth and differentiation, provides a mechanism for the E7 protein to affect cell proliferation. N. Dyson et al., Science 243: 934-936 (1989).

It is therefore a purpose of the present invention to provide synthetic peptides that inhibit the binding of the HPV E7 protein to the RBG protein. It is another purpose of this invention to provide methods for analyzing the binding inhibition activity of these synthetic peptides and other molecules in HPV E7 - RBG protein binding assays. A further purpose is to provide treatments of genital warts and cervical carcinoma utitizing these synthetic peptides and pharmaceutical compositions containing the synthetic peptides.

## SUMMARY OF THE INVENTION

The present invention provides a newly synthesised biochemically pure polypeptide (or polypeptides) which inhibits the binding of the HPV E7 protein to the RBG protein. This polypeptide was identified from assays of HPV E7 - RBG protein interaction. The invention also provides a method of using the polypeptide to treat genital warts and cervical carcinoma. The novel polypeptides of the present invention are useful in pharmaceutical compositions, as screening tools and in the prevention, prophylaxis, therapy and treatment of HPV induced diseases or other conditions which would benefit from inhibition of HPV infection.


DETAILED DESCRIPTION OF THE INVENTION

In one aspect, the invention is directed to a peptide of the following sequence identified from the noted regions of HPV-16:


Table A


Peptide A


Thr-Asp-Leu-Tyr-Cys-Tyr-Glu-Gln-Leu-Asn-Asp-Ser-Ser (representing residues 26-38 of HPV-16 E7 protein).

The data suggests that Peptide A is a single polypeptide of identified sequence, however, the possibility of homologs, isoforms or genetic variants of Peptide A exists either within or outside the cellular environment. This invention encompasses all such homologs, isoforms or genetic variants of Peptide A, provided each inhibits binding of HPV E7 protein to the RBG protein. Polypeptides that are homologs of Peptide A specifically include those having an amino acid sequence which is at least about 40% conserved in relation to the amino acid sequence set forth in Table A, preferentially at least about 60% conserved, and more preferentially at least about 75% conserved.

It will be understood by one of ordinary skill in the art that other variants of Peptide A are included within the scope of the present invention. This particularly includes any variants that differ from the synthesized Peptide A only by conservative amino acid substitution. Many such conservative amino acid substitutions are set forth as sets in Taylor, W.R., J. Mol. Biol. 188 , 233 (1986).

Peptide A, or fragments thereof, in this application includes any such variations in the amino acid sequence, whether by conservative amino acid substitution, deletion, or other processes, provided that the polypeptide after purification inhibits binding of the HPV E7 protein to the RBG protein. For example, Peptide C described in Example 3 is a derivative of Peptide A with 2 amino acids deleted. Peptide C retains the activity of Peptide A by inhibiting binding of the HPV E7 protein to the RBG protein.The fragments of Peptide A may be small peptides with sequences of as little as 4 or more amino acids, said sequences being those disclosed in Table A when said polypeptides inhibit the binding of the HPV E7 protein to the RBG protein.

Polypeptides larger than Peptide A are also included within the scope of the present invention when said polypeptides inhibit the binding of the HPV E7 protein to the RBG protein, and include a partial amino acid sequence as set forth in Table A, or conservative substitutions thereof.

It will be readily apparent to one of ordinary skill in the art that a great deal of use can be made of the amino acid sequence of Peptide A. For example, oligonucleotide probes can be constructed from the amino acid sequence and employed to screen for the cDNA clones encoding Peptide A. These clones containing Peptide A cDNA(s) can be used to transcribe mRNA which can then be translated and expressed. This work with Peptide A can be used to produce large quantities of Peptide A by genetic engineering or to study the genetics of Peptide A to learn its cellular role.

Additionally, synthetic polypeptides can be made in order to improve upon the pharmacological properties of Peptide A. These synthetic peptides can be made by the technique of solid-phase peptide synthesis discussed infra.

The amino acid sequence can also be used to make polypeptides which can then be used as a screen or tool for the identification of non-peptidal molecules which show an inhibitory effect on HPV E7 protein binding to the RBG protein.

The foregoing peptides may have various chemical modifications made at the terminal ends and still be within the scope of the present invention. In particular, the peptides may be prepared with or without an amide group at the C-terminus. When prepared with an amide group at its C-terminus, the peptides have a carboxy-terminal amide group covalently linked to the carbonyl moiety of the preceeding amino acid residue. The peptides can also have an acetyl group covalently attached to the amino acid at the N-terminus. Other chemical modifications are possible, particulary cyclic and dimeric configurations.

The polypeptides of the present invention find utility for the treatment of genital warts, cervical cancer or other conditions caused by HPV in man. The polypeptides of the present invention can be included in pharmaceutical compositions for the treatment or prevention of diseases involving HPV or the RBG protein as well as the other conditions discussed above.

In further aspects, the invention relates to the foregoing peptides conjugated to carriers capable of conferring immunogenicity on these peptides, to antisera raised against these peptides and the antibodies contained therein.

The peptides of the invention may be used to raise antibodies, either in subjects for which protection against infection by HPV is desired, i.e. as vaccines or to heighten the immune response to an HPV infection already present. They also can be injected into production species to obtain antisera. In lieu of the polyclonal antisera obtained in the production subjects, monoclonal antibodies may be produced using the standard methods or by more recent modifications thereof by immortalizing spleen or other antibody-producing cells for injection into animals to obtain antibody-producing clones. The polyclonal or monoclonal antibodies obtained, if corrected for species variations, can also be used as therapeutic agents.

Direct administration of the proteins to a host can confer either protective immunity against HPV or, if the subject is already infected, a boost to the subject's own immune response to more effectively combat the progress of the disease. For all applications, the peptides are administered in immunogenic form. Since the peptides are relatively short, this may necessitate conjugation with an immunogenicity conferring carrier material. This carrier material should ideally be antigenically neutral, i.e., ineffective in raising antibodies against itself. Antigenic neutrality is, of course, an ideal state as many carriers which are actually satisfactory do contain some antigenic regions which are capable of raising antibodies in the host. However, this may still be acceptable if the antigenic regions are in fact different from those of the peptide of interest, which is quite easy to achieve, or if the antibodies raised against the carrier portions are harmless to the subject.

The magnitude of a prophylactic or therapeutic dose of a polypeptide of this invention will, of course, vary with the group of patients (age, sex, etc.), the nature or the severity of the condition to be treated and with the particular polypeptide of this invention and its route of administration. In general, the daily dose range for use lies within the range of from about 0.5 TO ABOUT 5 Mg per kg body weight of a mammal.

Any suitable route of administration may be employed for providing a mammal, especially a human with an effective dosage of a polypeptide of this invention. For example, oral, rectal, vaginal, topical, parenteral, ocular, nasal, sublingual, buccal, intravenous and the like may be employed. Dosage forms include tablets, troches, dispersions, suspensions, solutions, capsules, creams, ointments, suppositories, aerosols and the like. Said dosage forms also include implanted slow releasing devices specifically designed for this purpose or other forms of implants modified to additionally act in this fashion.

The pharmaceutical compositions of the present invention comprise a polypeptide of this invention as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases including inorganic bases and organic bases. The compositions include compositions suitable for oral, rectal, ophthalmic, pulmonary, nasal, vaginal, sublingual, dermal, topical or parenteral (including subcutaneous, submucosol, intramuscular, intravenous and intra-arterial) administration, although the most suitable route in any given case will depend on the nature and severity of the conditions being treated and on the nature of the active ingredient. They may be conveniently presented in unit dosage form and prepared by any of the methods well-known in the art of pharmacy.

For administration by inhalation, the polypeptides of the present invention are conveniently delivered in the form of an aerosol spray presentation from pressurized packs or a nebuliser, or a powder which may be formulated as a cartridge from which the powder composition may be inhaled with the aid of a suitable device. The preferred delivery system for inhalation in a metered dose inhalation (MDI) aerosol, which may be formulated as a suspension or solution in fluorocarbon propellants.

Suitable topical formulations include transdermal devices, aerosols, creams, ointments, lotions, dusting powder, and the like.

In practical use, a polypeptide of this invention can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous and intra-arterial). In preparing the compositions for oral dosage form, any of the usual pharmaceutical media may be employed, such as, for example, water glycols, oils, alcohols, flavoring agents, preservatives, coloring agents and the like in the case of oral liquid preparations, such as, for example, suspensions, elixirs and solutions; or carriers such as starches, sugars, microcrystalline cellulose, diluents, granulating agents, lubricants, binders, disintegrating agents and the like in the case of oral solid preparations such as, for example, powders, capsules and tablets. Because of their ease of administration, tablets and capsules represent the most advantageous oral dosage unit form, in which case solid pharmaceutical carriers are obviously employed. If desired, tablets may be sugar coated or enteric coated by standard techniques.

In addition to the common dosage forms set out above, the polypeptides of this invention may also be administered by controlled release means and/or delivery devices.

Pharmaceutical compositions of the present invention suitable for oral administration may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent.

If the peptides are to be administered as vaccines, they are formulated according to conventional methods for such administration to the subject to be protected. If the antibodies are to be used for therapeutic purposes, it is generally desirable to confer species characteristics upon them compatible with the subject to be treated. Accordingly, it is often desirable to prepare these antibodies in monoclonal form since fusion with suitable partners is capable of conferring the desired characteristics on the secreted monoclonals.

Once designed, the peptides of the invention are prepared by any convenient means, commonly by chemical peptide synthesis using solid phase techniques. For conjugation to carrier protein, it is convenient to synthesize these peptides with an additional functionality, for example, a cysteine residue at the C-terminus to provide a convenient linkage. Depending on the nature of the linkers used, however, other approaches to form the conjugates are possible. The conjugated peptides are then administered to subject animals.

## Peptide Synthesis

As used herein, "peptide", "polypeptide", and "protein" are used interchangeably, and refer to amino acid sequences of a variety of lengths, either in their neutral (uncharged) forms or in forms which are salts, and either free of modifications such as glycosylation, side chain oxidation, or phosphorylation or containing these modifications. It is well understood in the art that amino acid sequences contain acidic and basic groups, and that the particular ionization state exhibited by the peptide is dependent on the pH of the surrounding medium when the protein is in solution, or that of the medium from which it was obtained if the protein is in solid form. Also included in the definition are proteins modified by additional substituents attached to the amino acid side chains, such as glycosyl units, lipids, or inorganic ions such as phosphates, as well as modifications relating to chemical conversions of the chains, such as oxidation of sulfhydryl groups. Thus, "peptide" or its equivalent terms is intended to include the appropriate amino acid sequence referenced, subject to those of the foregoing modifications which do not destroy its biological properties.

All of the peptides of the invention are sufficiently short that chemical synthesis, using methods now standard in the art, is feasible. See Merrifield ("Solid-Phase Peptide Synthesis", Advances in Enzymology, 32:221-296, 1969); G. Barnay & R.B. Merrifield "Solid-Phase Peptide Synthesis", The Peptides, Vol. 2, ed. E. Gross & 3. Merenhole (1980). This method is based on the strategy of having the carboxyl terminus of the peptide linked covalently to a solid support. The desired peptide sequence is prepared by stepwise coupling of single amino acids to a peptide chain growing from the carboxyl toward the amino terminus. Coupling is typically achieved by activation of the carboxyl group of the amino acid being attached to the resin which may have other potentially reactive groups blocked. Following addition of an amino acid to the growing polypeptide chain, and prior to further chain elongation, a protecting group is typically removed. Because each amino acid is coupled by nearly the same series of reactions, the need for elaborate strategies in the synthesis is minimized. Solubility is not a major issue during synthesis, because the peptide is linked to a solid support. This method is rapid and it can be utilized simply. It is very convenient for the synthesis of multiple analogs with amino-terminal substitutions, because a single synthesis can be branched in multiple directions near the amino terminus, thereby creating many analogs varying only in the amino terminal region.

Recombinant DNA methodology provides an alternative way of synthesizing the desired peptides. The DNA coding sequence for the desired peptide or protein is ligated into an expression vector suitable for transforming a recipient cell, which is thus caused to express the gene and produce the protein. The DNA coding sequences are sufficiently short to be prepared synthetically using means known in the art.

The coding sequence is placed under the control of control sequences compatible with recombinant hosts in plasmids containing convenient restriction sites for insertion of the desired coding sequence. Alternatively, these peptides can be produced in bacterial or nonbacterial, i.e., yeast, recombinant hosts using appropriate control sequences, vectors and transformation techniques.

In order to identify antagonists of the HPV E7 protein - RBG protein interaction, the amino acid sequence of the HPV-16 E7 protein was examined. The amino acid sequence of the HPV-16 E7 protein is depicted in Table B.

## Table B

### Amino acid sequence of the HPV-16 E7 protein.

```
      1    2    3    4    5    6    7    8    9   10   11   12   13   14   15
NH2-Arg-Asn-Pro-Ala-Val-Ile-Met-His-Gly-Asp-Thr-Pro-Thr-Leu-His-

    16                                                           31
Glu-Tyr-Met-Leu-Asp-Leu-Gln-Pro-Glu-Thr-Thr-Asp-Leu-Tyr-Cys-Tyr-

    32                                                           47
Glu-Gln-Leu-Asn-Asp-Ser-Ser-Glu-Glu-Glu-Asp-Glu-Ile-Asp-Gly-Pro-

    48                                                           63
Ala-Gly-Gln-Ala-Glu-Pro-Asp-Arg-Ala-His-Tyr-Asn-Ile-Val-Thr-Phe-

    64                                                           79
Cys-Cys-Lys-Cys-Asp-Ser-Thr-Leu-Arg-Leu-Cys-Val-Gln-Ser-Thr-His-

    80                                                           95
Val-Asp-Ile-Arg-Thr-Leu-Glu-Asp-Leu-Leu-Met-Gly-Thr-Leu-Gly-Ile-

    96                          104
Val-Cys-Pro-Ile-Cys-Ser-Gln-Lys-Pro-COOH
```

### Peptide A contains residues number 26 through 38.
### Peptide B contains residues number 46 through 61.

Several distinct regions of this protein were selected to provide the sequence of peptides that might interfere with binding of the E7 protein to the RBG protein. The sequence of two of the peptides that were synthesized and tested are underlined in Table B. The peptides corresponding to these underlined sequences were synthesized and isolated as described in Example 1. The purified peptides were then assayed for their ability to inhibit binding of the HPV E7 protein to the RBG protein as described in Example 2.

An additional peptide unrelated to the HPV E7 protein was also tested for its ability to inhibit binding of the HPV E7 protein to the RBG protein. This additional peptide consisted of the last eight amino acid residues of the human gastrin releasing peptide (Acetyl-GRP 20-27). This GRP peptide is described in Heimbrook et al., J. Biol. Chem. 263: 7016-7019 1988.

Only Peptide A inhibited the binding of the HPV E7 protein to the RBG protein. Neither Peptide B with the following sequence:
Gly-pro-Ala-Gly-Gln-Ala-Glu-Pro-Asp-Arg-Ala-His-Tyr-Asn-Ile-Val

nor the Acetyl-GRP 20-27 peptide inhibited the binding of the HPV E7 protein to the RBG protein. These experiments indicate that a peptide of the sequence Thr-Asp-Leu-Tyr-Cys-Tyr-Glu-Gln-Leu-Asn-Asp-Ser-Ser-NH$_2$ has the ability to block binding of the HPV E7 protein to the RBG product. These experiments further show that other peptides identified from different regions of the E7 protein or from other proteins can not inhibit HPV E7 protein binding to the RBG protein.

The following examples illustrate the present invention without limiting the same thereto.

EXAMPLE 1

SYNTHESIS OF HPV E7 PEPTIDES

The peptides representing different regions of the HPV-16 E7 protein were synthesized. The sequence of these peptides was taken from the amino acid sequence of distinct segments of the HPV-16 E7 protein as indicated in Table B. The peptides are referred to as Peptide A and Peptide B. Peptide A has the sequence:

Thr-Asp-Leu-Tyr-Cys-Tyr-Glu-Gln-Leu-Asn-Asp-Ser-Ser-NH$_2$

where the amide group attached to the serine residue at position 13 represents a carboxy-terminal amide group covalently linked to the carbonyl moiety of the serine residue.

Peptide B has the sequence:

Gly-Pro-Ala-Gly-Gln-Ala-Glu-Pro-Asp-Arg-Ala-His-Tyr-Asn-Ile-Val

where the valine residue at position 16 was incorporated as the carboxylate.

Peptide A corresponds to residues 26-38 of the HPV-16 E7 protein. Peptide B corresponds to residues 46-61 of the HPV-16 E7 protein. These peptides were synthesized using the techniques of solid phase peptide chemistry as described by R. B. Merrifield, J. Am. Chem. Soc. 85: 2149-2154 1963, and J. M. Stewart and J. D. Young in Solid Phase Peptide Synthesis, Pierce Chem. Co., Rockeford, Ill., 2d ed., 1984. The actual syntheses were performed on an Applied Biosystems Model 430A peptide synthesizer. Peptide A was synthesized on a p-methyl-benzhydrylamine resin. Use of this resin permits isolation of carboxy terminally amidated peptides following cleavage with hydrogen fluoride. Peptide B was synthesized on an amino acyl-phenylacetamidomethyl resin. Use of this resin permits isolation of peptides with a free carboxylic acid group at the carboxy terminal amino acid residue. Both peptides were synthesized using commercially available BOC amino acids possessing protected side chain groups. Cleavage and deprotection of the resin-bound peptide was achieved by treatment with liquid hydrogen fluoride containing scavengers. Purification of the cleaved peptides was performed by reversed phase high performance liquid chromatography (HPLC) using a Vydac C-18 column. The method of purification using HPLC was as described by J. Rivier et al. J. Chromatogr. 288: 303-328 1984. Once isolated, the composition of each peptide was validated by amino acid analysis using a Beckman 6300 amino acid analyzer (Beckman Instruments, Palo Alto, California).

EXAMPLE 2

BINDING INHIBITION ACTIVITY OF SYNTHETIC PEPTIDES

Peptide A, Peptide B, and Acetyl-GRP 20-27 were assayed for their abilities to inhibit the binding of the HPV-16 E7 protein to the RBG protein. Binding inhibition assays were carried out in vitro using a modification of the procedure described by N. Dyson et al., Science 243: 934-936 1989. Whole cell lysates of Hela cells were used as a source of human RBG protein. Rabbit reticulocyte cell free lysates that permit transcription and translation of exogenously added DNAs served as a source of HPV-16 E7 protein. The HPV-16 E7 gene was added to the rabbit reticulocyte lysate as a recombinant DNA clone under the control of a T7 promoter and incubated for 60 minutes at 30°C in the presence of radiolabeled methionine to allow transcription of the HPV-16 E7 gene and translation into E7 protein that contained radiolabeled methionine. The Hela cell and rabbit reticulocyte lysates were then mixed and incubated at 4°C for 90 minutes. After further manipulations a murine antibody against the RBG protein was added and incubated at 4°C for 60 minutes. Next, a rabbit anti-mouse IgG antibody and protein A sepharose was added to the reaction mixture in order to immune precipitate the RBG protein. At the same time, the E7 protein that was bound to the RBG protein was also precipitated. After an additional 60 minute incubation at 4°C the precipitated proteins were isolated by centrifugation and examined by polyacrylamide gel electrophoresis. Following electrophoresis, the polyacrylamide gel was dried and exposed to x-ray film. Since the HPV-16 E7 protein used in these reactions was radiolabelled during its synthesis, the x-ray film reveals the presence of E7 protein that was bound to the RBG protein and therefore immune precipitated along with the RBG protein using the anti-RBG protein antibody. This biochemical protocol yields a quantitative estimate of the binding of HPV-16 E7 protein to the RBG protein in vitro .

These biochemical reactions can be run with or without added synthetic peptides to evaluate the effects of various concentrations of peptides on the binding of the HPV E7 protein to the RBG protein. In our studies, the addition of peptide A to these reaction mixtures at concentrations of 40 uM, 20 uM, or 2 uM caused a reduction in HPV-16 E7 protein binding to the RBG protein of 90%, 90% and 60% respectively. Addition of Peptide B or Acetyl-GRP 20-27 to the reaction mixture at concentrations up to 60 uM had no effect on HPV-16 E7 protein binding to the RBG protein.


EXAMPLE 3


DERIVATIVE OF PEPTIDE A THAT BLOCKS BINDING OF HPV E7 PROTEIN TO THE RBG PROTEIN

A synthetic peptide, designated Peptide C, was synthesized using the techniques described in Example 1. The sequence of Peptide C is as follows:
Ac-Leu-Tyr-Cys-Tyr-Glu-Gln-Leu-Asn-Asp-Ser-Ser-NH$_2$
where Ac indicates an acetyl group covalently attached to the alpha amine moiety of the leucine residue, and NH$_2$ represents an amide group covalently attached to the carbonyl moiety of the serine residue.

Peptide C is a derivative of Peptide A described in Example 1. Therefore, the sequence of Peptide C is similar to the sequence of Peptide A and includes the amino acid residues of the HPV-16 E7 protein numbers 28 through 38. Peptide C differs from Peptide A by having the amino acid residues number 26 and 27 deleted and by having an acetyl group covalently attached to the leucine residue at position 28.

Peptide C was examined in the assay described in Example 2. Peptide C inhibited HPV-16 E7 protein binding to the RBG protein at concentrations of Peptide C as low as 1 uM. This experiment shows that derivatives of Peptide A that encompass segments of Peptide A smaller than the entire 13 amino acids residues of Peptide A are inhibitors of HPV E7 protein binding to the RBG protein. Additionally, Peptide C is itself an antagonist of the RPV E7 binding to the RBG protein.


Example 4


E7/RB Binding Assays:

An in vitro solution assay used for the detection of complex formation between E7 protein and p105-RB has been described by Dyson, et al (30) . Cell lysates used as a source of RBG protein were prepared from human T24 bladder carcinoma cells as described (30) . C36 and the control antibody Pb416 are monoclonal antibodies directed against RGB protein and SV40 large T protein, respectively. These antibodies were a generous gift from E. Harlow (Cold Spring Harbor Laboratories, Cold Spring Harbor, NY). Immunoprecipitates of E7/RBG protein binding reactions containing serially diluted peptide antagonists were dissolved in 35 μl gel loading buffer (30) . Five μl was counted directly in Right Safe (Beckman, Palo Alto, CA.) scintillation cocktail and 25 μl was subjected to sodium dodecyl sulphate poly-acrylamide gel electrophoresis. Gels were dried and exposed to Kodak X-AR film at -70°C overnight. In order to more rapidly assay large numbers of samples and more easily quantitate the binding results, we developed a

30. Dyson, N., Duffy, L.A., and Harlow, E. (1989( Cancer Cells 1 , 235-240.

plate binding assay for the detection of E7/RBG protein association. Purified recombinant E7 was resuspended in 0.1 M sodium carbonate, pH 9.5, at 1 ng/$\mu$l. Fifty microliters of this solution was placed into each well of a polyvinyl chloride microtiter plate (COSTAR 2596, Cambridge, MA.). The plate was gently agitated for 18 hours at 4°C. The E7 solution was aspirated off and 100 $\mu$l of plate blocking buffer (PBB) [50mM Hepes 7.0, 250 mM sodium chloride, 0.1% NP4O, 0.5% (w/v) pigskin gelatin, and 0.02% sodium azide] was added per well. The buffer was removed and the plate was washed three times in the above buffer without the gelatin (PB). Five femtomoles of $^{35}$S-methionine (Amersham 1100 Ci/mmole, Arlington Heights, IL.) labeled RBG protein prepared in a rabbit reticulocyte lysate was diluted in PB buffer containing various peptide antagonists to a final volume of 50 $\mu$l and added to the E7 treated plate. The plate was again incubated at 4°C with agitation for 1 hour after which the solution was aspirated and each well washed five times with PB. The top of the plate was removed using a hot wire cutting apparatus (D. Lee, Sunnyvale, CA.). Each well was placed in 3 ml of Ready Safe scintillation cocktail (Beckman, Palo Alto, CA.) and counted for 1 minute.

## Example 5

### Peptide Syntheses:

Peptides (see Table C) were prepared by solid-phase synthesis (31), using a double coupling protocol for the introduction of all amino acids on the model 430A Applied Biosystems automated peptide synthesizer. Deprotection and removal of the peptide from the resin support was achieved by treatment with liquid hydrofluric acid (32). The peptides were purified by preparative high-pressure liquid chromatography (HPLC) on reverse phase C18 silica columns using an aqueous 0.1% trifluoroacetic acid-acetonitrile gradient (33). Homogeneity of the peptides was demonstrated by analytical HPLC and identity was confirmed by amino acid composition analysis.

Table C lists twenty-three peptides prepared using these techniques. These peptides include homologs of peptide A that inhibit E7 binding to the RBG protein.

31. Merrifield, R.B. (1963) J. Am. Chem. Soc. 85, 2149-2154

32. Sakakibara, S., Shimonishi, Y., Kishida, Y., Okada, M., and Sugihara, H. (1967) Bull. Chem. Soc. Japan 40, 2164-2167

33. Rivier, J. McClentock, R., and Anderson, H. (1984) J. Chromatogr. 288, 303-328

TABLE C

| PEPTIDE ANTAGONISTS OF E7/RBG PROTEIN ASSOCIATION | | |
|---|---|---|
| PEPTIDES | SOLUTION ASSAY RBG Protein/E7 $IC_{50}$ | PLATE ASSAY RBG Protein/E7 $IC_{50}$ |
| 1. E7-(20-32)-AMIDE | 1.0 | 0.05 |
| 2. E7-(20-30)-AMIDE | 1.0 | 0.08 |
| 3. E7-(20-29)-AMIDE | 1.0 | 0.09 |
| 4. E7-(20-28)-AMIDE | 5.0 | 0.70 |
| 5. [Gln27]]-E7-(20-27)-AMIDE | 50.0 | 9.10 |
| 6. E7-(20-26)-AMIDE | 100.0 | 3.30 |
| 7. E7-(14-29)-AMIDE | ND | 0.08 |
| 8. E7-(18-29)-AMIDE | 0.4 | 0.06 |
| 9. E7-(20-29)-AMIDE | 1.0 | 0.09 |
| 10. E7-(21-32)-AMIDE | 20.0 | 11.40 |
| 11. E7-(20-29)-ACID | ND | 0.08 |
| 12. N-Ac-E7-(20-29)-ACID | ND | 0.06 |
| 13. N-Ac-E7-(20-29)-AMIDE | ND | 0.08 |
| 14. N-Ac-E7-(21-29)-AMIDE | 1.0 | 0.04 |
| 15. N-Ac-E7-(22-32)-AMIDE | 10.0 | 25.00 |
| 16. [Ser$^{24}$]-E7-(20-32)-AMIDE | 40.0 | ND |
| 17. [ABU$^{24}$]-E7-(20-32)-AMIDE | 20.0 | ND |
| 18. [ACM$^{24}$]-E7-(20-32)-AMIDE | > 100.0 | ND |
| 19. [Phe$^{23}$]-E7-(20-29)-AMIDE | 1.0 | 0.60 |
| 20. [Phe$^{25}$]-E7-(20-29)-AMIDE | 15.0 | > 50.00 |
| 21. [Gln$^{26}$]-E7-(20-28)-AMIDE | > 100.0 | ND |
| 22. [Asn$^{21}$]-E7-(20-28)-AMIDE | > 100.0 | ND |
| 23. [Asn$^{27}$]-E7-(20-30)-AMIDE | 1.0 | ND |

$IC_{50}$ values represent the average of at least two experiments in which each concentration of inhibitor was tested in triplicate. The variation between experiments was less than 10%. The 50% inhibition concentrations are micromolor. ND = not determined.

**Claims**

1. A polypeptide having an amino acid sequence
Leu-Tyr-Cys-Tyr-Glu-Gln-Leu-Asn-Asp-Ser-Ser
which inhibits binding of the HPV E7 protein to the RBG protein.
2. A polypeptide having the formula
ThrAsp-Leu-Tyr-Cys-Tyr-Glu-Gln-Leu-Asn-Asp-Ser-Ser;
or
Leu-Tyr-Cys-Tyr-Glu-Gln-Leu-Asn-Asp-Ser-Ser.
3. A polypeptide of Claim 2 which further contains an amide group at its C-terminus.
4. A polypeptide of Claim 3 which further contains an acetyl group at its N-terminus.
5. A polypeptide of Claim 2 which is
Thr-Asp-Leu-Tyr-Cys-Tyr-Glu-Gln-Leu-Asn-Asp-Ser-Ser-NH2;
or
Ac-Leu-Tyr-Cys-Tyr-Glu-Gln-Leu-Asn-Asp-Ser-Ser-NH$_2$.
6. A polypeptide which comprises one or more polypeptides each of which is an inhibitor of the HPV E7 protein binding to the RBG protein and has a partial amino acid sequence of
Leu-Tyr-Cys-Tyr-Glu-Gln-Leu-Asn-Asp-Ser-Ser
or conservative substitutions thereof.
7. A fragment of the polypeptides of Claim 6 which comprises small peptides with sequences of 4 or more amino acids, said sequences being those of the polypeptides of Claim 6.

8. The use of a polypeptide according to Claim 1 for the preparation of a medicament useful for treating genital warts in a mammal.

9. The use of a polypeptide according to Claim 1 for the preparation of a medicament useful for treating cervical cancer in a mammal.

10. A method of identifying molecules which are inhibitors of the HPV E7 protein binding to the RBG protein which comprises:

(a) contacting said molecule with a polypeptide of Claim 1; and

(b) measuring the effect of HPV E7 protein binding to he RBG protein relative to the effect in the absence of said molecule.

11. A pharmaceutical composition for treating genital warts or cervical cancer which comprises a therapeutically effective amount of a polypeptide according to Claim 1 and a pharmaceutically acceptable carrier.

12. A polypeptide having the amino acid sequence:

E7-(20-32)-AMIDE;

E7-(20-30)-AMIDE;

E7-(20-29)-AMIDE;

E7-(20-28)-AMIDE;

[Gln27]]-E7-(20-27)-AMIDE;

E7-(20-26)-AMIDE;

E7-(14-29)-AMIDE;

E7-(18-29)-AMIDE;

E7-(20-29)-AMIDE;

E7-(21-32)-AMIDE;

E7-(20-29)-ACID;

N-Ac-E7-(20-29)-ACID;

N-Ac-E7-(20-29)-AMIDE;

N-Ac-E7-(21-29)-AMIDE;

N-Ac-E7-(22-32)-AMIDE;

[Ser24]-E7-(20-32)-AMIDE;

[ABU24]-E7-(20-32)-AMIDE;

[ACM24]-E7-(20-32)-AMIDE;

[Phe23]-E7-(20-29)-AMIDE;

[Phe25]-E7-(20-29)-AMIDE;

[Gln26]-E7-(20-28)-AMIDE;

[Asn21]-E7-(20-28)-AMIDE; or

[Asn27)-E7-(20-30)-AMIDE.